# EUROPEAN PATENT APPLICATION

(11) **EP 3 483 785 A1**
(43) Date of publication of application: **15.05.2019**
(21) Application number: 18204249.9
(22) Date of filing: 05.11.2018
(51) Int. Cl.: G06K 9/00, A61B 5/16

(54) **SYSTEM AND METHOD FOR GUIDING SOCIAL INTERACTIONS**

(30) Priority: 09.11.2017 US 201715807688
(71) Applicant: General Electric Company, Schenectady, NY 12345 (US)
(72) Inventor: TU,, Peter Henry, Niskayuna, NY New York 12309 (US); GAO,, Tao, Niskayuna, NY New York 12309 (US); TU,, Jilin, Schenectady, NY New York 12309 (US)
(74) Representative: Serjeants LLP

(57) **Abstract**

A social interaction system (10) and of operation thereof for interpreting social cues and using such analysis to improve social interaction via the providing of a suggested response is provided. The social interaction system (10) includes one or more sensors (12) to obtain social indicator data of one or more individuals (14) in an environment (16) during a social interaction, the social indicator data related to a behavior of the one or more individuals. The social interaction system (10) also includes a processing system (28) configured to determine a social state of the one or more individuals (14) using the social indicator data and determine an optimal social response of a person to the social interaction based on an analysis of the social state. The social interaction system (10) further includes a feedback system (136) to indicate the optimal social response to the person.

## Description

### BACKGROUND OF THE INVENTION

Embodiments of the invention relate generally to interpreting and analyzing social interaction and, more particularly, to a method and apparatus for interpreting social cues and using such analysis to improve social interaction via the providing of a suggested response.

The ability of an individual to recognize common social cues is understood to be a fundamental building block of engaging and interacting with others in an effective and meaningful manner. However, for various reasons and in numerous circumstances, it is recognized that the ability of an individual to recognize social cues may be hindered or otherwise made more difficult, whether it be due to differences between the individuals communicating (e.g., cultural differences) or due to a condition of one of the individuals.

A primary example of the ability to recognize social cues is with individuals living with Autism Spectrum disorder (ASD). That is, some individuals with ASD have trouble recognizing social cues displayed by others during a social interaction, such as not perceiving social meaning behind facial expressions and body language, and thus such individuals can have difficulty interacting socially with other people. An individual who does not recognize social cues is unlikely to respond to the cues appropriately, which could negatively impact the social interaction.

An important coping mechanism for individuals with ASD involves learning to recognize common social cues so that they can respond to the social situation appropriately. For instance, a high functioning autistic person may have learned to recognize when a joke has been told during a social interaction. The person may not find the joke to be humorous, but has learned that social etiquette often requires laughter in response to a joke. The high functioning autistic person could benefit from assistance identifying social cues while developing such coping mechanisms for social interaction.

While some individuals with ASD can learn different coping strategies, other individuals with ASD lack the innate ability to recognize certain social cues. For instance, some individuals with ASD have difficulty recognizing paralinguistic cues while others may have difficulty distinguishing facial expressions or body language. When the innate ability to recognize certain social cues is absent, an individual may not be able to develop coping strategies related to those cues. Individuals who lack the innate ability to recognize certain social cues could benefit from assistance identifying the social cues during social interaction.

Many individuals can recognize common social cues but could use help identifying subtle social cues or social cues from a different culture. For instance, an individual may be engaged in a social interaction with many people but fail to discern important social cues from one person when focusing on others. In addition, a person may be engaged in a social interaction with people of a foreign culture and may not understand common social cues of that culture. Aid in identifying social cues could help an individual respond appropriately to those cues to improve social interaction.

Therefore, it would be desirable to design an apparatus and method to improve social interaction.

### BRIEF DESCRIPTION OF THE INVENTION

The invention is directed to a method and apparatus for interpreting social cues and using such analysis to improve social interaction via the providing of a suggested response.

In accordance with one aspect of the invention, a social interaction system includes one or more sensors to obtain social indicator data of one or more individuals in an environment during a social interaction, the social indicator data related to a behavior of the one or more individuals. The social interaction system also includes a processing system configured to determine a social state of the one or more individuals using the social indicator data and determine an optimal social response of a person to the social interaction based on an analysis of the social state. The social interaction system further includes a feedback system to indicate the optimal social response to the person.

The processing system may use reinforcement learning to determine the optimal social response, with the processing system configured to:
determine the optimal social response using a policy to map the social state to the optimal social response,
determine whether the optimal social response results in a desirable social outcome of the social interaction; and
update the policy to optimize a future social response based on an analysis of the social outcome.

The processing system may use domain knowledge to determine the optimal social response by mapping the social state to the optimal social response using a policy that has predefined relationships between mapped social states and social responses.

The feedback system may operate in real-time.

The feedback system may comprise a wearable augmented reality system.

The one or more sensors may comprise at least one camera or at least one microphone.

The one or more sensors may comprise at least one wearable sensor.

The processing system may use a probabilistic inference engine to determine the social state based on the social indicator data by inferring a social state estimated to result in the behavior of the one or more individuals.

The social state may be a group social state determined by the probabilistic inference engine based on group behavioral statistics.

The processing system may be a wearable processing device.

In accordance with another aspect of the invention, a system for assisting a user with social interaction includes one or more sensors to obtain data indicating social expression of one or more individuals and a processing system programmed to extract social cues from the social expression data and determine a social response based on the social cues to assist the user interact socially with the one or more individuals, the social response determined by applying a predefined policy based upon social outcomes. The system also includes a feedback system to indicate the social response to the user.

In determining the social response, the processing system may be programmed to apply the predefined policy using domain knowledge by mapping the social cues to the social response using a predefined relationship between mapped social cues and social responses.

In determining the social response, the processing system may be programmed to apply the predefined policy using machine learning based upon:
a state space determined from the social cues,
an action space to determine the social response from the state space, and
a reward based on a social outcome of the user interacting socially with the one or more individuals resulting from the social response; and
wherein the predefined policy optimizes the social response by maximizing the reward.

The one or more sensors may comprise an audio, visual, or physiological sensor.

The processing system may further comprise an inference engine to determine a social state based on the social cues, wherein the predefined policy maps the social state to the social response.

The feedback system may be a wearable feedback system.

In accordance with yet another aspect of the invention, a non-transitory computer readable storage medium having stored thereon a computer program for optimizing social outcomes is disclosed, the computer program comprising instructions that, when executed by a processor, cause the processor to retrieve data of one or more persons involved in a social interaction using one or more sensors, extract social cues from the social interaction using the data, estimate a social state based on the social cues of the one or more persons involved in the social interaction, and map the social state to a suggested action of a person to engage in the social interaction using a policy that optimizes suggested actions based on social outcomes.

The policy may have a predefined relationship between social states and suggested actions.

The policy may optimize future suggested actions based upon previous social outcomes.

The data may be retrieved from at least one of an audio sensor or visual sensor.

The data may indicate verbal or non-verbal social expression.

The policy may optimize future suggested actions based upon delayed rewards.

The instructions may further cause the processor to provide the suggested action to a feedback system.

The feedback system may operate in a forensic feedback mode.

Various other features and advantages will be made apparent from the following detailed description and the drawings.

### BRIEF DESCRIPTION OF THE DRAWINGS

The drawings illustrate preferred embodiments presently contemplated for carrying out the invention.

In the drawings:
FIG. 1 is a pictorial view of a social interaction system having wall mounted sensors, in accordance with an embodiment of the invention.
FIG. 2 is a pictorial view of a social interaction system worn by an individual, in accordance with an embodiment of the invention.
FIG. 3 is a flowchart illustrating a technique performed by a social interaction system to aid an individual with social interaction, in accordance with an embodiment of the invention.

### DETAILED DESCRIPTION

The operating environment of the invention is described with respect to a social interaction system used by an individual to improve social interaction. However, it will be appreciated by those skilled in the art that the invention is equally applicable for use by other people seeking information related to a social interaction. Moreover, the invention will be described with respect to a wearable or a non-wearable social interaction system. However, one skilled in the art will further appreciate that the invention is equally applicable to a social interaction system that includes components from both the wearable and the non-wearable systems.

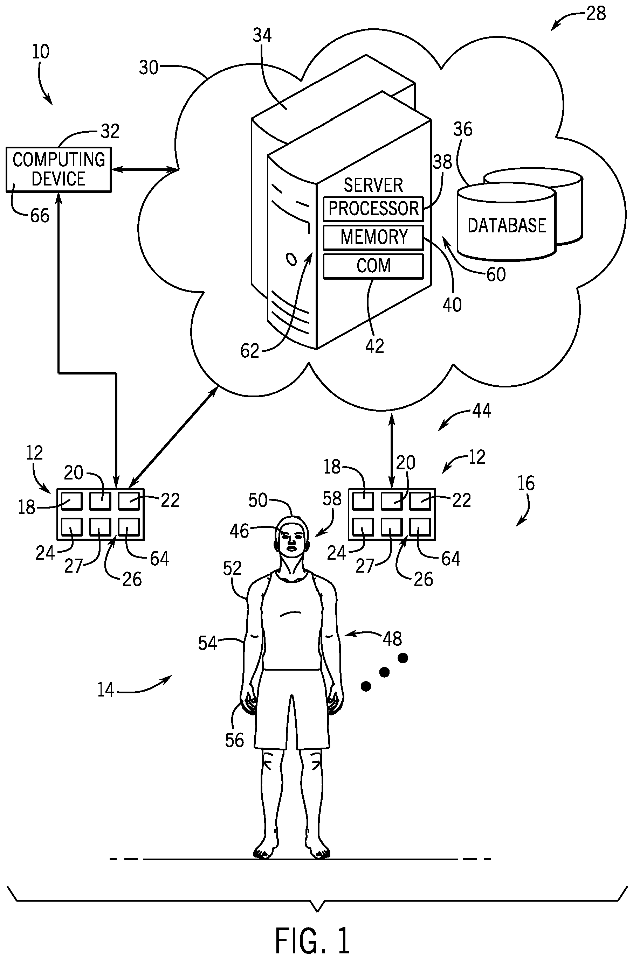

Referring to FIG. 1, a social interaction system 10 to assist a person with social interaction is shown, in accordance with an embodiment of the invention. The social interaction system 10 preferably uses one or more sensors 12 to obtain social indicator data of one or more individuals 14 in an environment 16 during a social interaction. The one or more sensors 12 may include an audio 18, visual 20, physiological, or any other sensor to obtain social indicator data related to a behavior of the one or more individuals 14. The social indicator data may be used to interpret visual, audible, or physiological cues from individuals 14 or groups of individuals involved in a social interaction. The social interaction system 10 can help a person participate in social interaction by automatically interpreting social cues and provide feedback regarding the cues to the individual in real-time. For example, the social interaction system 10 may be tailored to promote a transition to independence for individuals living with ASD.

In one embodiment, the sensors 12 are preferably non-invasive standoff sensors 22 so that the social interaction system 10 obtains social indicator data from one or more individuals 14 who are not instrumented with sensors. The sensors 12 may be installed in any suitable location of the environment 16, and could be mounted to a wall, ceiling, floor, door, window, furniture, or the like. The environment 16 that is instrumented may be a room in a facility that has frequent social interactions of the type a user the system seeks to improve. For instance, classrooms or common areas in assisted living facilities may be instrumented with microphones 24 and cameras 26 to capture social cues from common social interactions occurring in those facilities. In other embodiments, the social interaction system 10 obtains social indicator data from one or more individuals 14 who are instrumented with sensors.

The sensors 12 are shown in FIG. 1 as cameras 26 enabled to capture video, still images, or both. An environment 16 may be instrumented with multiple cameras 26 for multi-angle tracking and to easily track all persons 14 in the environment 16. In one embodiment, a site may be instrumented with multiple cameras 26 capable of multi-person tracking of groups up to 6 individuals or more. The cameras 26 may include pantilt-zoom (PTZ) cameras 27 tasked with capturing high resolution images of all tracked individuals 14. The cameras 26 may be instrumented with microphones 24 to capture audio data from people 14 in the environment 16.

The sensors 12 may be communicatively coupled to transmit data to a processing system 28. The embodiment of FIG. 1 shows the sensors 12 coupled to transmit data to a cloud-based computing system 30. The embodiment of FIG. 1 also shows the sensors 12 coupled to transmit data to a computing device 32 located in the environment 16 with the sensors 12. However, the sensors 12 could be coupled to transmit or receive data from only one of the computing device 32 and the cloud-based computing system 30, or another computing system. Either of the cloud-based computing system 30 or the computing device 32 may be programmed to receive data from the cameras 26 at preprogrammed intervals (e.g., every 1 second, 10 seconds, 30 seconds, 1 minute, 10 minutes, 1 hour, or 1 day), or either may be programmed to receive data from the sensors 12 in real-time or near real-time.

The cloud-based computing system 30 may include one or more servers 34 and one or more databases 36 located externally from the servers. Each server 34 may include one or more processors 38 and one or more memories 40. The social indicator data may be received by the one or more servers 34 and stored on the one or more memories 40 or the one or more databases 36. Each server 34 may also have a communications component 42 to facilitate wired or wireless communications between the servers 34, databases 36, computing device 32, and/or the sensors 12. The servers 34 may communicate with each other to distribute tasks between each other to be performed more efficiently.

The processor 38 may be one or more computer processors or microprocessors capable of executing computer-executable code. The computer-executable code may be stored on the memory 40 which may comprise any suitable non-transitory media that can store processor-executable code used by the processor 38 to perform the presently disclosed techniques. The memory 40 may be any suitable type of computer-readable media that can store the processor-executable code, data, analysis of the data, or the like. The database 36 may also be a computer-readable non-transitory storage medial capable of storing processor-executable code, data, analysis of the data, or the like. The memory 40 and/or the database 36 may store cognitive models used by the processor 38 to execute behavior recognition analysis. A history of the data received from the sensors 12 or data processed by the processor 38 may be stored on the memory 40 or database 36.

The processor 38 generally analyzes a behavior from the one or more individuals 14 using data from the sensors 12. The processor 38 unit may be programmed to automatically detect social signals using the sensors 12 and use a variety of social analytics to extract social cues of the social interaction. For instance, the processor 38 may acquire signals from the sensors 12 related to the social interaction and use the data to extract visual and audible cues from people 14 involved in the interaction. The processor 38 may run various applications stored on the memory 40 to process the social indicator data, and the processor 38 may be updated with the most recent advances in situational awareness methods. The processor 38 may store an analysis of the social indicator data on the memory 40, on the database 36, and/or output the analysis to the computing device 32.

Audio signals captured by the sensors 12 may be analyzed to extract semantically meaningful expression and paralinguistic cues including sarcasm, gasps, laughter, or other audible cues. The social interaction system 10 may use natural language processing to extract verbal cues captured by the sensors 12. Voice to text can be used to detect semantically meaningful expressions, utterances, words, phrases or other verbal cues. Machine learning can be applied to audio signals captured by the sensors 12 to compute paralinguistic cues including sarcasm, gasps, laughter, and other audible cues. In one embodiment, the social interaction system 10 analyzes a behavior of an individual 14 using the processor 38 to perform speech recognition.

Visual signals captured by the sensor 12 may be analyzed to extract visual cues including facial expression, gaze direction, body motion, body gestures, body posture, and/or location of individuals 14, or other visual cues. The social interaction system 10 may use computer vision (CV) technologies 44 to interpret visual cues from a social interaction. The computer vision technologies 44 include one or more cameras 26 to capture data representing visual signals from a social interaction and processes the data to extract visual cues. For instance, visual or other social signals from individuals 14 or groups of individuals can be analyzed using the GE Sherlock system by General Electric Corp. of Boston, Massachusetts.

The processor 38 may use a variety of computer vision algorithms to extract social expression data. For instance, the social indicator data may be processed using computer vision algorithms to provide expression recognition or other facial analysis. The computer vision algorithms may comprise one or more social interaction modules that can be selectively incorporated into the social analysis system 10. The modules may include one or more of a tracking and proximity module, an affective pose and gestures module, a gaze analysis module, and eyeball 46 analysis module, and a facial expression module.

Regarding the tracking and proximity module, a detect-and-track paradigm may use range cameras or red, green, blue, and depth (RGB+D) cameras to generate a set of person detections. Foreground motion detection and sliding window classifiers produce a set of possible person detections on a per frame basis. By comparing frames, the set of possible person detections may be associated with a set of person trackers that can be used to produce a ground plane trajectory for each person 14. Measures including proximity, speed, stationarity, or other measures can be extracted from the person trackers.

Regarding the affective pose and gestures module, given a high-resolution image of the upper body 48, affective pose can be extracted by detecting the positions of the head 50, shoulder 52, elbows 54 and hands 56. Detecting these positions results in a type of skeleton model of the upper body 48. By analyzing the temporal evolution of the upper body skeleton model, social cues including specific pose positions and certain social gestures can be detected, among others. Machine learning methods such as Deep Learning can be used to extract skeleton models on a frame by frame basis.

Regarding the gaze analysis module, given a high-resolution image of the face 58, a facial landmark model can be fitted to the face using both generative and discriminative methods. The shape of the facial landmark model can be used to estimate the 3D pose position of the face 58 relative to a camera 26 capturing the image. The 3D pose position can be used as a proxy for gaze direction.

Regarding the eyeball 46 analysis module, given a facial landmark model, individual eyeball regions can be identified. Image processing techniques that model the spatial distribution of white regions of the eye 46 can be used to detect eyeball motion. Eyeball 46 motion can be used to detect social cues including furtive glances or social acts including power staring, among others.

Regarding the facial 58 expression module, given a facial landmark model, a rectified facial image can be generated. Discriminative methods (e.g., Deep Learning methods) can be used to classify the rectified facial images resulting in the recognition of various facial 58 expressions.

The processing system 28 may include one or more inference engines 60 to determine a social state of people 14 involved in the social interaction based on the extracted social cues. The inference engine 60 may be stored on the memory 40 and executed by the processor 38. The inference engines 60 may automatically analyze the social expression data using computer algorithms to compute social states including joy, frustration, hostility, excitement, anger, fear, surprise, or any other social state. The inference engines 60 may determine the social state of an individual 14 interacting with the user including the state of rapport, levels of mutual trust, or any other social variable. The inference engines 60 may be Bayesian (probabilistic) inference engines 62 and may be based on generative or discriminative modeling techniques to infer the current social state of a given social interaction.

For instance, the processing system 28 may use a probabilistic inference engine 62 to determine the social state based on the social indicator data by inferring a social state estimated to result in the behavior of the one or more individuals 14. The inference engines 60 may also estimate group behavioral statistics including levels of group rapport and levels of group trust. That is, the social state may be a group social state determined by the probabilistic inference engine 62 based on group behavioral statistics. As will be explained further below, the inference engines 60 can establish a set of social states based on the social cues which can be used to develop a coping strategy for ASD individual hoping to participate in group level interactions.

In one embodiment, the inference engines 60 may utilize an inference approach involving the use of forward simulation where artificial agents that model various cognitive processes are used to mirror the observed behaviors resulting in an interpretation of the cognitive states of each individual as well as group level cognitive states. Accordingly, visual or other social signals may be analyzed using techniques described in U.S. Patent Application Serial No. 15/370,736 filed December 6, 2016, the disclosure of which is incorporated herein by reference in its entirety.

Regarding group level interactions, the social interaction system 10 may be based on a set of computer vision technologies 44 that can automatically interpret non-verbal social interactions of groups of individuals 14 using non-invasive stand-off sensors 22 including ceiling mounted range imagers 64 and an array of wall mounted PTZ cameras 27. Upon entering an instrumented site, all individuals 14 maybe tracked. PTZ cameras 27 may automatically target the faces 58 of all individuals 14. Facial expressions, gaze directions, body motions, and/or group level dynamics may be extracted in real-time. For instance, a stream of data representing person 14 specific cues may be distilled into a set of site-level aggregate statistics that are independent of the number or configuration of people 14 observed. A set of social signals may then be computed including positive/negative effect, physical activity, engagement, mimicry, or any other social signal. Bayesian inference engines 62 may be used to determine the state of various social variables including group rapport, levels of mutual trust, or other social variables.

Detected behavior of the one or more people 14 may be displayed to a user using the computing device 32, which may serve as a graphical user interface (GUI) 66 for the social interaction system 10. The computing device 32 may be a desktop computer, laptop computer, tablet, smartphone, smartwatch, or other computing device. The computing device 32 may provide a front-end display of data or results obtained from the cloud-based computing system 30. For instance, the computing device 32 may access an internet website which displays information received from the cloud-based computing system 30. The computing device 32 may also control the social interaction system 10. For instance, the computing device 32 may have computer applications stored thereon to control or program the sensors 12, and/or the cloud-based computing system 30. In other embodiments the computing device 32 may execute functions of the one or more servers 34 and/or the one or more databases 36 to operate the social interaction system 10 without the cloud-based computing system 30.

The computing device 32 may also provide feedback to a user of the social interaction system 10 regarding detected social cues or social states. The feedback may be in the form of visual, audible, of physical indications provided to the user. The computing device 32 may provide feedback to help a person interact with other people 14 or to interact with one or more computers (e.g., computing device 32) or other electronic devices. In one embodiment, the computing device 32 helps a person interact with a smartphone which may indicate social cues detected during interpersonal communication. In another embodiment, the person living with ASD could receive feedback from another person having direct access to the computing device 32. Knowledge gained from the computing device 32 could be used to develop a coping strategy for individuals with ASD seeking to improve social interaction.

In addition to operating in real-time for recognition and guidance, the computing device 32 may operate in a forensic feedback mode. The forensic feedback mode may provide a synopsis of a social interaction. The synopsis could include a visual summary of the encounter, an interpretation of the observed social cues/social states, and reasoning behind any conclusions drawn. Exposure to coaching using the forensic feedback mode may increase the ability of an individual with ASD to independently recognize subtle social cues/social states. The forensic feedback may be used to gain understanding of various human social phenomena, leading to insight regarding how an ASD individual can best cope with complex and poorly understood interactions.

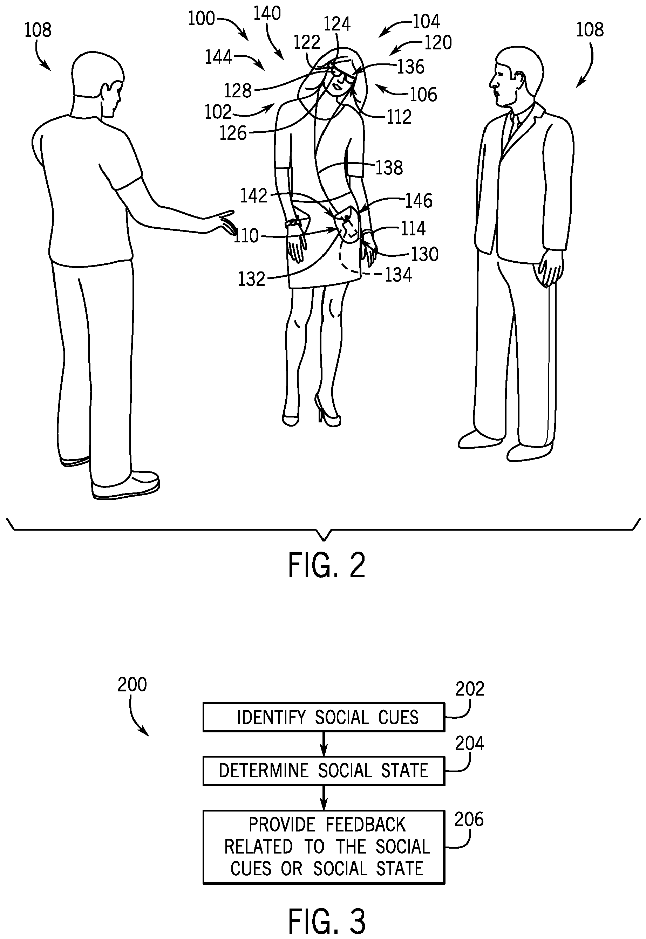

Referring now to FIG. 2, a social interaction system 100 having wearable components 102 is shown, in accordance with an embodiment of the invention. The social interaction system 100 preferably assists a user 104 with social interaction. While FIG. 1 shows a social interaction system 10 that is not worn by a person using the system, FIG. 2 shows a social interaction system 100 that is an entirely person borne device. Alternatively, the social interaction system 100 may include some components that are person 104 borne while other components are located remotely from the person. Any components 102 of the social interaction system 100 could be connected to each other in order to transmit and/or receive data including connections by wired link or wireless link, for example via Bluetooth® or WiFi®.

The social interaction system 100 may include one or more sensors 106 to obtain data indicating social expression of one or more individuals 108. The data may be retrieved from at least one of an audio sensor 110, visual sensor 112, or physiological sensor 114. The data can indicate verbal or non-verbal social expression from one or more persons 108 involved in a social interaction. For instance, computer vision algorithms can be applied to video feeds to extract a plethora of social cues including facial expressions, gaze directions, eye movements (e.g., averted glances), upper body affective pose, upper body gestures, and/or other social cues. The sensors 106 may be wearable sensors 120, non-wearable sensors, or a combination of wearable and non-wearable sensors. For example, sensors may be worn by an individual or installed onsite at a location of the social interaction. In one embodiment, the one or more sensors may comprise at least one wearable sensor 120.

Wearable sensors 120 may allow an individual to travel while using the social interaction system 100. The one or more sensors 106 may include at least one camera 122, at least one microphone 124, or at least one physiological sensor 114 wearable by the user 104 or by another person 108. A camera 122 and/or microphone 124 could be part of a body camera device worn by an individual 104. Alternatively, a wearable sensor device 120 could be mounted to a set of eye-glasses 126 having an optical head-mounted display 128. For instance, the social interaction system 100 may use a wearable device 120 similar to Google Glass™ by Google Inc. of Mountain View, California. The wearable device 120 may detect social signals from people 108 interacting with the user 104 but could also detect social signals from the user 104. For instance, physiological signals captured by physiological sensors 114 may be analyzed to extract physiological cues including blood pressure, heart rate, or other physiological cues.

The processing system 130 may be a wearable processing device 132 which is shown in FIG. 2 as a smartphone 134 worn by an individual 104. The processing system 130 preferably executes one or more computer algorithms to analyze data received from the sensors 106 and provide an output of data to a feedback system 136. The processing device 132 may be integrated with sensors or a feedback system as a single component. For instance, the smartphone 134 may have a sensor including a microphone, a camera, and/or an accelerometer. The smartphone 134 may also have a feedback system including a display, a speaker, and/or a vibration device.

The feedback system 136 may be a wearable feedback system and is shown in FIG. 2 as a wearable augmented reality system. The wearable augmented reality system 136 operates in real-time and may include smartglasses 126 to provide a heads-up display 128 and earphones 138 to provide audible signals. The feedback may be in the form of words, symbols, pictures, tones, vibrations, amplification, enhancement, or any other suitable form of indication. The feedback could provide identification of detected social cues/social states of one or more individuals 108 involved in a social interaction (an empathy aid) or it could provide instructions or guidance to act in a way that produces a desired social outcome (a coach).

The social interaction system 100 can operate as an empathy aid 140 to help individuals 104 interpret social cues and/or identify social states from people 108 interacting socially. In one embodiment, the empathy aid 140 captures data related to a social interaction using the sensors 106, extracts social cues or social states using the processor 132, and indicates the social cues or social states to a person 104 using the feedback system 136. The feedback system 136 may be an augmented reality assistant 142 that amplifies verbal or non-verbal social cues in real-time for an individual 104 seeking to improve a social outcome. The empathy aid 140 may interpret social cues and identify social states of individuals or groups of individuals 108. For instance, the empathy aid 140 may indicate social cues or social states from multiple people 108, or indicate group social cues and/or group social states. The empathy aid 140 may provide a fully automatic device to help people living with ASD better understand social interactions. For example, a group of people as a whole may have become exasperated with a particular user of the social interaction system 100. In such situations perilous group level actions may ensue without early indication of a hostile group level social state from the social interaction system 100.

The social interaction system 100 can operate as an oracle 144 to coach individuals 104 to respond to social cues or social states from people 108 interacting socially. In one embodiment, the oracle 144 may provide a suggested action in response to social cues or social states in real-time to a person 104 seeking to improve the outcome of a social interaction. The oracle 144 captures data related to a social interaction using the sensors 106, determines the suggested action using the processor 132, and indicates the suggested action to a person 104 using the feedback system 136. For instance, the processing system 130 may be configured to determine a social state of one or more individuals 108 using social indicator data and determine an optimal social response of a person 104 to the social interaction based on an analysis of the social state.

The suggested action provided by the oracle 144 may be in the form of instructions, commands, or signals. For instance, the suggestion action may instruct the user 104 to display social cues to other people 108 involved in the social interaction (e.g., smile, wave, laugh, speak, etc). The oracle 144 may also incorporate the empathy aid 140 and thereby indicate social cues or social states to the person 104. The suggested action may be a response to group level social interaction. For instance, the suggested action may be a response to social cues or social states from multiple people 108, or from group social cues and/or group social states. The feedback system 136 may indicate the social response to the user 104. The oracle 144 may provide a fully automatic device to help people living with ASD better interpret and respond to social interactions. In addition, the oracle 144 could also provide feedback to a person who is trying to work with a person living with ASD such as an employer, teacher, caregiver, or any other person interacting socially with the person having ASD.

It is recognized that social situations can become awkward if an expected reaction to social cues is absent, and thus a person living with ASD could use the oracle 144 to tell them when a social situation expects certain behavior, such as laughing after a joke has been told. A high functioning autistic person may develop a rule that they will laugh whenever a joke has been told, even if that person does not find the joke to be humorous. For people living with ASD, the oracle 144 may automatically indicate the presence of social cues and that a response to the cues is anticipated. In one embodiment, the oracle 144 may use sensors 106 to detect when verbal or non-verbal social cues indicate a joke has been told, inference engines 146 to determine social states are appropriate for laughter, and provide feedback to instruct a person with ASD to begin laughing.

In operating to coach individuals 104 to respond to social cues or social states, the oracle 144 may use a predefined policy to map social cues or social states to a social response suggested to a user 104 to improve a social interaction. The social response may be determined by applying a predefined policy based upon social outcomes. The policy may be generated based on domain knowledge, machine learning, or any other technique capable of generating the policy. Domain knowledge and machine learning both may offer an automated method to map inputs like social cues or social states to suggested actions. For example, a processing system 130 may be programmed to extract social cues from social expression data, and determine a social response based on the social cues to assist the user 104 interact socially with the one or more individuals 108. Domain knowledge may suggest predefined actions mapped to common social cues, while machine learning may optimize suggested actions based on prior social outcomes. In some embodiments, the policy may be based on a combination of domain knowledge and machine learning.

A policy based on domain knowledge may have a predefined relationship between social states and suggested actions. According to one embodiment, in determining the social response, the processing system 130 may be programmed to apply a predefined policy using domain knowledge by mapping the social cues to the social response using a predefined relationship between mapped social cues and social responses. A feedback system 136 may be used to indicate the optimal social response to a person 104. According to another embodiment, the processing system 130 may use domain knowledge to determine the optimal social response by mapping a social state to the optimal social response using a policy that has predefined relationships between mapped social states and social responses.

A policy based on machine learning may drive the policy to optimize future suggested actions based upon previous social outcomes. Machine learning may include learned frameworks such as reinforcement learning (RL) which can generate and optimize the policy over time and can also optimize long term social outcomes. The learning framework can also be used to enhance detection of social outcomes to determine whether a desired outcome has taken place. For instance, the processing system 130 may use reinforcement learning to determine the optimal social response, with the processing system configured to determine the optimal social response using a policy to map the social state to the optimal social response, determine whether the optimal social response results in a desirable social outcome of the social interaction, and update the policy to optimize a future social response based on an analysis of the social outcome.

In addition to crafting a policy based on a specific person's experiences, the experiences of multiple users can be aggregated in a reinforcement learning paradigm so that each individual may benefit from a policy developed based on multiple experiences of multiple individuals. Accordingly, machine learning need not be restricted to a specific user 104 but could be based on a large number of users. In this way, a general policy could be learned based on the experiences/outcomes of all users such that each user may benefit from the collective experiences of a community of users.

The learning framework can update the policy based on the extent to which prior social outcomes were positive or negative, and can also update the policy based on social states including emotional states resulting from the interaction. For example, a person with ASD interacts with a co-worker and at the end of the interaction the emotional state of the co-worker may be estimated using facial expressions. If the estimated emotional state is positive, then the interaction may be considered to have a positive outcome. If the social outcome was not optimal, the policy could update to indicate a different suggested action in the future. An updated policy may provide a suggestion to the user 104 to be more contrite in a future response. The best policy for suggested actions may be learned over time based on both observed social states and outcomes of the interaction.

The reinforcement learning framework for policy generation is preferably based on state spaces, action spaces, and rewards. The state space includes the inferred social states based on the detected social cues. The action space includes feedback provided to the user 104 to improve social outcomes. The reward includes positive outcomes of the social interaction. In determining the social response, the processing system 130 may be programmed to apply the predefined policy using machine learning based upon a state space determined from the social cues, an action space to determine the social response from the state space, and a reward based on a social outcome of the user 104 interacting socially with the one or more individuals 108 resulting from the social response. The predefined policy may optimize the social response by maximizing the reward. The combination of the state space, action space, and reward provides a learning based approach to define a policy that will increase the likelihood of a positive social outcome.

The social interaction system 100 can be tailored for specific sub-populations. The policy can be created to target a certain sub-population that will benefit the most out of a population. For example, a high functioning autistic person may be grouped into a sub-population of persons living with ASD. A high functioning autistic person may find they have developed rules by trial and error regarding their future response to reoccurring social cues. The social interaction system 100 may automate rule development for immediate implementation rather than by trial and error.

In some embodiments, the policy may be tailored to account for different types of users 104 as well as different types of people 108 interacting with the user. For instance, the policy may be generated specifically for police, soldiers, salesmen, or people living with ASD.

As one example, the social interaction system 100 may be tailored to aid soldiers interacting with foreign civilian populations. While interacting socially with foreign civilian populations, soldiers can have difficulty interpreting and responding appropriately to cultural specific verbal or non-verbal social cues. Soldiers may be able to improve social interactions with foreign civilian populations using an empathy aid 140 to indicate social cues to the soldier or an oracle 144 to guide the soldier responding to foreign interactions. The social interaction system 100 can also measure psychosocial factors used to determine the degree to which a soldier has acquired the skills required to interact with foreign civilian populations.

As another example, the social interaction system 100 may be tailored to aid salespersons interacting with customers. An empathy aid 140 could aid a salesperson by amplifying social cues or an oracle 144 could aid a salesman by coaching the salesperson through a sale. The oracle 144 may use a learning framework based upon a reward such as actions taken by the customer. For example, facial images could be captured of a customer while interacting with a salesperson who is using an oracle 144 to guide a sales pitch. Another facial image could be captured of the customer at a point-of-sale linking the customer to the interaction. A positive outcome to the interaction could be determined if information from the point-of-sale indicates that an item sold relates to a suggested action from the oracle 144. The purchase of an item constitutes a reward that is delayed from the suggested action, and therefore the policy may optimize future suggested actions based upon delayed rewards.

As yet another example, the social interaction system 100 may be tailored to aid caregivers interacting with patients. A caregiver (e.g. nurse, doctor, etc.) often must establish a sense of empathy or trust with a patient to obtain their cooperation in receiving care. For instance, a patient may resist care, like taking medication or receiving a painful procedure, without first establishing trust or empathy with the caregiver. Caregivers may use an empathy aid 140 to determine whether the patient is demonstrating social cues consistent with perceived trust or empathy, and could also use an oracle 144 to guide the caregiver in reaching a state of trust or empathy with the patient. The social interaction system 100 may also be used to establish rapport with the patient to ensure care is given in a manner that achieves a positive result. In addition, a patient could use the social interaction system 100 to determine whether the caregiver is demonstrating certain social cues or social states. For instance, the patient may use the empathy aid 140 to determine that the caregiver is demonstrating social cues indicating trust and empathy, or an oracle 144 to help guide the patient reach a state of trust and empathy with the caregiver.

Referring now to FIG. 3, and with continued reference back to FIGS. 1 and 2, a flow diagram of a process 200 used to indicate feedback to a person seeking to improve social interaction is illustrated, in accordance with an embodiment of the invention. The process 200 begins at step 202 by identifying social cues from people involved in a social interaction. The social cues may be identified via data collected from one or more sensors that obtain data indicating social expression of one or more individuals, which may be sensors 12 positioned in an environment and/or worn by an individual 104. The data can indicate verbal or non-verbal social expression from one or more persons involved in a social interaction, including facial expressions, gaze directions, eye movements (e.g., averted glances), upper body affective pose, upper body gestures, and/or other social cues that are indicative of a behavior or emotional state of the individual.

Upon the identification of social cues from people involved in a social interaction, the process 200 continues at step 204 by determining a social state of people involved in the social interaction, with the social state determined based on the social cues. That is, one or more computer algorithms may function to analyze the data received from the sensors and the identified social cues to extract and determine a social state therefrom. The determination of the social state includes interpreting social cues of individuals or groups of individuals to determine the social state, with possible social states of an individual including joy, frustration, hostility, excitement, anger, fear, or surprise, for example. Additionally, the determination of the social state may extend to the social state between the individuals, including the state of rapport, level of mutual trust, etc.

The determination of the social state of the people involved in the social interaction allows for identification of appropriate next steps to further the social interaction between the individuals. The process 200 thus continues at step 206 by providing feedback related to the social cues or social state to the person seeking to improve the social interaction. In one embodiment, the system operates as an empathy aid to indicate and relay extracted social cues or social states to a person 104 using the feedback system 136. As one example, an augmented reality assistant 142 may amplify verbal or non-verbal social cues in real-time for an individual 104 seeking to improve a social outcome, including social cues/states from one or multiple people, including group social cues and/or social states. In another embodiment, the system operates as an oracle 144 to coach an individual 104 on how to respond to social cues or social states from people 108 interacting socially, such as by providing a suggested action in response to social cues or social states in real-time to the person. That is, the oracle 144 may determine an optimal social response of a person 104 to the social interaction based on an analysis of the social state, with the analysis being performed via a predefined policybased on domain knowledge, machine learning, or any other technique capable of generating the policy -- to map social cues or social states to a social response suggested to a user to improve a social interaction. The oracle 144 may then provide a suggested action in the form of instructions, commands, or signals, such as by instructing the person 104 to display social cues to other people 108 involved in the social interaction, e.g., to smile, wave, laugh, speak, etc.

A technical contribution for the disclosed method and apparatus is that it provides for a computer implemented method of extracting social cues from a social interaction and of providing feedback regarding the social cues to a person 104 seeking to improve social interaction. In one embodiment, a non-transitory computer readable storage medium has stored thereon a computer program for optimizing social outcomes. The computer program may include instructions that, when executed by the processor 132, causes the processor to retrieve data of one or more persons 108 involved in a social interaction using one or more sensors 106 and extract social cues from the social interaction using the data. In another embodiment, a computer program may comprise instructions that, when executed by a processor, cause the processor to estimate a social state based on the social cues of the one or more persons 108 involved in the social interaction, and map the social state to a suggested action of a person 104 to engage in the social interaction using a policy that optimizes suggested actions based on social outcomes. The instructions may cause the processor 132 to provide the suggested action to a feedback system 136.

One skilled in the art will appreciate that embodiments of the invention may be interfaced to and controlled by a computer readable storage medium having stored thereon a computer program. The computer readable storage medium includes a plurality of components such as one or more of electronic components, hardware components, and/or computer software components. These components may include one or more computer readable storage media that generally stores instructions such as software, firmware and/or assembly language for performing one or more portions of one or more implementations or embodiments of a sequence. These computer readable storage media are generally non-transitory and/or tangible. Examples of such a computer readable storage medium include a recordable data storage medium of a computer and/or storage device. The computer readable storage media may employ, for example, one or more of a magnetic, electrical, optical, biological, and/or atomic data storage medium. Further, such media may take the form of, for example, floppy disks, magnetic tapes, CD-ROMs, DVD-ROMs, hard disk drives, and/or electronic memory. Other forms of non-transitory and/or tangible computer readable storage media not listed may be employed with embodiments of the invention.

A number of such components can be combined or divided in an implementation of a system. Further, such components may include a set and/or series of computer instructions written in or implemented with any of a number of programming languages, as will be appreciated by those skilled in the art. In addition, other forms of computer readable media such as a carrier wave may be employed to embody a computer data signal representing a sequence of instructions that when executed by one or more computers causes the one or more computers to perform one or more portions of one or more implementations or embodiments of a sequence.

Beneficially, the social interaction system may provide a social signal amplification system that increases the capacity of an individual living with ASD to integrate into society. The social interaction system may also incorporate computer vision technologies with an augmented reality assistant for the purposes of interpreting and responding to individual or group level social interactions. The social interaction system may also increase the likelihood of obtaining positive social outcomes by mapping social cues to suggested actions using a policy based on domain knowledge or machine learning. The social interaction system may also measure social cues and improve the detection of social outcomes.

A technical effect of the methods, systems, and apparatus described herein includes a computer implemented technique for interpreting social cues and using such analysis to improve social interaction via the providing of a suggested response.

Therefore, according to one embodiment of the invention, a social interaction system includes one or more sensors to obtain social indicator data of one or more individuals in an environment during a social interaction, the social indicator data related to a behavior of the one or more individuals. The social interaction system also includes a processing system configured to determine a social state of the one or more individuals using the social indicator data and determine an optimal social response of a person to the social interaction based on an analysis of the social state. The social interaction system further includes a feedback system to indicate the optimal social response to the person.

According to another embodiment of the invention, a system for assisting a user with social interaction includes one or more sensors to obtain data indicating social expression of one or more individuals and a processing system programmed to extract social cues from the social expression data and determine a social response based on the social cues to assist the user interact socially with the one or more individuals, the social response determined by applying a predefined policy based upon social outcomes. The system also includes a feedback system to indicate the social response to the user.

According to yet another embodiment of the invention, a non-transitory computer readable storage medium having stored thereon a computer program for optimizing social outcomes is disclosed, the computer program comprising instructions that, when executed by a processor, cause the processor to retrieve data of one or more persons involved in a social interaction using one or more sensors, extract social cues from the social interaction using the data, estimate a social state based on the social cues of the one or more persons involved in the social interaction, and map the social state to a suggested action of a person to engage in the social interaction using a policy that optimizes suggested actions based on social outcomes.

This written description uses examples to disclose the invention, including the best mode, and also to enable any person skilled in the art to practice the invention, including making and using any devices or systems and performing any incorporated methods. The patentable scope of the invention is defined by the claims, and may include other examples that occur to those skilled in the art. Such other examples are intended to be within the scope of the claims if they have structural elements that do not differ from the literal language of the claims, or if they include equivalent structural elements with insubstantial differences from the literal languages of the claims.

## Claims

1. A social interaction system comprising:
one or more sensors to obtain social indicator data of one or more individuals in an environment during a social interaction, the social indicator data related to a behavior of the one or more individuals;
a processing system configured to:
determine a social state of the one or more individuals using the social indicator data; and
determine an optimal social response of a person to the social interaction based on an analysis of the social state; and
a feedback system to indicate the optimal social response to the person.

2. A social interaction system according to claim 1, wherein the processing system uses reinforcement learning to determine the optimal social response, with the processing system configured to:
determine the optimal social response using a policy to map the social state to the optimal social response,
determine whether the optimal social response results in a desirable social outcome of the social interaction; and
update the policy to optimize a future social response based on an analysis of the social outcome.

3. A social interaction system according to claim 1, wherein the processing system uses domain knowledge to determine the optimal social response by mapping the social state to the optimal social response using a policy that has predefined relationships between mapped social states and social responses.

4. A social interaction system according to any preceding claim, wherein the one or more sensors comprises at least one camera or at least one microphone, preferably wherein the one or more sensors comprises at least one wearable sensor.

5. A social interaction system according to any preceding claim, wherein the processing system uses a probabilistic inference engine to determine the social state based on the social indicator data by inferring a social state estimated to result in the behavior of the one or more individuals.

6. A social interaction system according to claim 5, wherein the social state is a group social state determined by the probabilistic inference engine based on group behavioral statistics.

7. A system for assisting a user with social interaction, the system comprising:
one or more sensors to obtain data indicating social expression of one or more individuals;
a processing system programmed to:
extract social cues from the social expression data, and
determine a social response based on the social cues to assist the user interact socially with the one or more individuals, the social response determined by applying a predefined policy based upon social outcomes; and
a feedback system to indicate the social response to the user.

8. A system according to claim 7, wherein, in determining the social response, the processing system is programmed to apply the predefined policy using domain knowledge by mapping the social cues to the social response using a predefined relationship between mapped social cues and social responses.

9. A system according to claim 7, wherein, in determining the social response, the processing system is programmed to apply the predefined policy using machine learning based upon:
a state space determined from the social cues,
an action space to determine the social response from the state space, and
a reward based on a social outcome of the user interacting socially with the one or more individuals resulting from the social response; and
wherein the predefined policy optimizes the social response by maximizing the reward.

10. A system according to any of claims 7 to 9, wherein the processing system further comprises an inference engine to determine a social state based on the social cues; and
wherein the predefined policy maps the social state to the social response.

11. A non-transitory computer readable storage medium having stored thereon a computer program for optimizing social outcomes, the computer program comprising instructions that, when executed by a processor, cause the processor to:
retrieve data of one or more persons involved in a social interaction using one or more sensors;
extract social cues from the social interaction using the data;
estimate a social state based on the social cues of the one or more persons involved in the social interaction; and
map the social state to a suggested action of a person to engage in the social interaction using a policy that optimizes suggested actions based on social outcomes.

12. A non-transitory computer readable storage medium according to claim 11, wherein the policy has a predefined relationship between social states and suggested actions or wherein the policy optimizes future suggested actions based upon previous social outcomes.

13. A non-transitory computer readable storage medium according to claim 11 or claim 12, wherein the data indicates verbal or non-verbal social expression.

14. A non-transitory computer readable storage medium according to any of claims 11 to 13, wherein the policy optimizes future suggested actions based upon delayed rewards.

15. A non-transitory computer readable storage medium according to any of claims 11 to 14, wherein the instructions further cause the processor to provide the suggested action to a feedback system, preferably wherein the feedback system operates in a forensic feedback mode.
